# EUROPEAN PATENT APPLICATION

(11) **EP 2 048 495 A1**
(43) Date of publication of application: **15.04.2009**
(21) Application number: 07791768.0
(22) Date of filing: 01.08.2007
(51) Int. Cl.: G01N 27/416, G01N 27/333

(54) **ELECTROLYTE ANALYZER AND ITS MEASURED DATA PROCESSING METHOD**

(30) Priority: 02.08.2006 JP 2006211389
(71) Applicant: Olympus Corporation, Tokyo 151-0072 (JP)
(72) Inventor: ISHIBE, Isao, Shibuya-ku, Tokyo 151-0072 (JP)
(74) Representative: von Hellfeld, Axel
(86) International application number: PCT/JP2007/065088
(87) International publication number: WO 2008/016087

(57) **Abstract**

An electrolyte analyzing apparatus including an A/D converter (45) which converts analogue data output from an ion selective electrode into digital data, a data processor (46a) which calculates a median of multiple pieces of the digital data converted from multiple pieces of the analogue data output from the ion selective electrode and outputs the median as measurement data of an electrolyte, and a calculator (46b) which calculates a concentration of the electrolyte contained in a specimen based on the measurement data of the electrolyte output from the data processor; and a measurement data processing method of the same.

## Description

### TECHNICAL FIELD

The present invention relates to an electrolyte analyzing apparatus and a measurement data processing method of the same.

### BACKGROUND ART

Conventionally, as an electrolyte analyzing apparatus which analyzes an electrolyte contained in a specimen such as a urine, a blood, and the like, an electrolyte analyzing apparatus which uses an ion selective electrode to measure a concentration of an electrolyte has been known (see Patent Document 1, for example). The electrolyte analyzing apparatus uses the ion selective electrode and a comparison electrode, and measures an electromotive force (a potential difference between a potential detected by the ion selective electrode and a potential detected by the comparison electrode) of a diluted specimen, which is a specimen diluted by a diluting liquid and an electromotive force of a reference liquid to determine a concentration of the electrolyte contained in the specimen based on measurement data of the diluted specimen and the reference liquid.

Patent Document 1: Japanese Patent Application Laid-Open No. 2001-4586

### DISCLOSURE OF INVENTION

### PROBLEM TO BE SOLVED BY THE INVENTION

The conventional electrolyte analyzing apparatus sometimes has an irregular noise which arises unexpectedly as a pulsing voltage in a measurement signal output from the ion selective electrode and the comparison electrode due to an unexpected electrical or mechanistic cause. Data including such an unexpected irregular noise results in an error and cannot be used as measurement data of the specimen. Therefore, the electrolyte analyzing apparatus would require once again measuring the specimen corresponding to the data with the pulsing noise and take a long time for measuring the specimen, which has led to a demand for taking measures against such an irregular noise.

The present invention has been achieved in view of the foregoing, and an object of the present invention is to provide: an electrolyte analyzing apparatus which can avoid an unexpected irregular noise contained in measurement data of a specimen, and can precisely measure the specimen without a waste of time; and a measurement data processing method of the same.

### MEANS FOR SOLVING PROBLEM

To solve the problem described above and achieve the object, an electrolyte analyzing apparatus according to the present invention includes: an A/D converter which converts analogue data output from an ion selective electrode into digital data; a data processor which calculates a median of multiple pieces of the digital data converted from multiple pieces of the analogue data output from the ion selective electrode and outputs the median as measurement data of an electrolyte; and a calculator which calculates a concentration of the electrolyte contained in a specimen based on the measurement data of the electrolyte output from the data processor.

In the electrolyte analyzing apparatus according to the present invention as set forth in the invention described above, the A/D converter employs a delta-sigma conversion system.

In the electrolyte analyzing apparatus according to the present invention as set forth in the invention described above, the ion selective electrode is provided in plural number for each electrolyte as a measurement subject, and the A/D converter receives the analogue data output from each ion selective electrode in a time division manner.

To solve the problem described above and achieve the object, a measurement data processing method of the electrolyte analyzing apparatus according to the present invention, the measurement data processing method being a measurement method of calculating a concentration of an electrolyte contained in a specimen based on digital data converted from analogue data output from an ion selective electrode, wherein a median of multiple pieces of the digital data converted from multiple pieces of the analogue data output from the ion selective electrode is treated as the measurement data of the electrolyte.

In the measurement method of calculating the concentration of the electrolyte according to the present invention as set forth in the invention described above, the digital data is obtained by performing a delta-sigma A/D conversion on the analogue data.

### EFFECT OF THE INVENTION

In the electrolyte analyzing apparatus according to the present invention, the data processor calculates a median of multiple pieces of digital data converted from multiple pieces of analogue data output from the ion selective electrode and outputs the median as measurement data of an electrolyte and the calculator calculates a concentration of the electrolyte contained in a specimen based on the measurement data. In a measurement data processing method in the electrolyte analyzing apparatus according to the present invention, the median of multiple pieces of digital data converted from multiple pieces of analogue data output from the ion selective electrode is treated as the measurement data of the electrolyte. Hence, the electrolyte analyzing apparatus and the measurement data processing method have an advantageous effect that the specimen can be precisely measured without a waste of time while an unexpected irregular noise contained in measurement data of the specimen is avoided.

### BRIEF DESCRIPTION OF DRAWINGS

FIG. 1 is a schematic diagram of a structure of an electrolyte analyzing apparatus according to an embodiment of the present invention;
FIG. 2 is a detail view of a control unit of the electrolyte analyzing apparatus shown in FIG. 1;
FIG. 3 shows a measurement data processing method in which multiple pieces of digital data is sorted and a median of the digital data is treated as measurement data of an electrolyte by a data processor of the control unit shown in FIG. 2; and
FIG. 4 is a flowchart showing one example of the measurement data processing method, executed under a control of the control unit, of the electrolyte analyzing apparatus.

### EXPLANATIONS OF LETTERS OR NUMERALS

- 1: SPECIMEN SUPPLYING UNIT
- 2: SPECIMEN VESSEL
- 3: DUCT
- 4: SPECIMEN DISPENSING NOZZLE
- 10: REFERENCE LIQUID SUPPLYING UNIT
- 11: REFERENCE LIQUID VESSEL
- 12: DUCT
- 13: ELECTROMAGNETIC VALVE
- 14: SYRINGE PUMP
- 15: ELECTROMAGNETIC VALVE
- 16: DISPENSING NOZZLE
- 20: DILUTING LIQUID SUPPLYING UNIT
- 21: DILUTING LIQUID VESSEL
- 22: DUCT
- 23: ELECTROMAGNETIC VALVE
- 24: SYTINGE PUMP
- 25: ELECTROMAGNETIC VALVE
- 26: DISPENSING NOZZLE
- 30: MEASURING UNIT
- 31: DILUTION TANK
- 32: DUCT
- 33: MEASUREMENT CELL
- 34: PUMP
- 35: COMPARISON ELECTRODE LIQUID VESSEL
- 36: DUCT
- 37: ELECTROMAGNETIC VALVE
- 38: STIRRING BAR
- 40: CONTROL UNIT
- 41 to 44: PREAMPLIFIERS
- 45: A/D CONVERTER
- 46: PROCESSING CONTROLLER
- 46a: DATA PROCESSOR
- 46b: CALCULATOR
- 47: COMMUNICATION INTERFACE
- 51: INPUT UNIT
- 52: DISPLAY UNIT
- 53: OUTPUT UNIT

### BEST MODE(S) FOR CARRYING OUT THE INVENTION

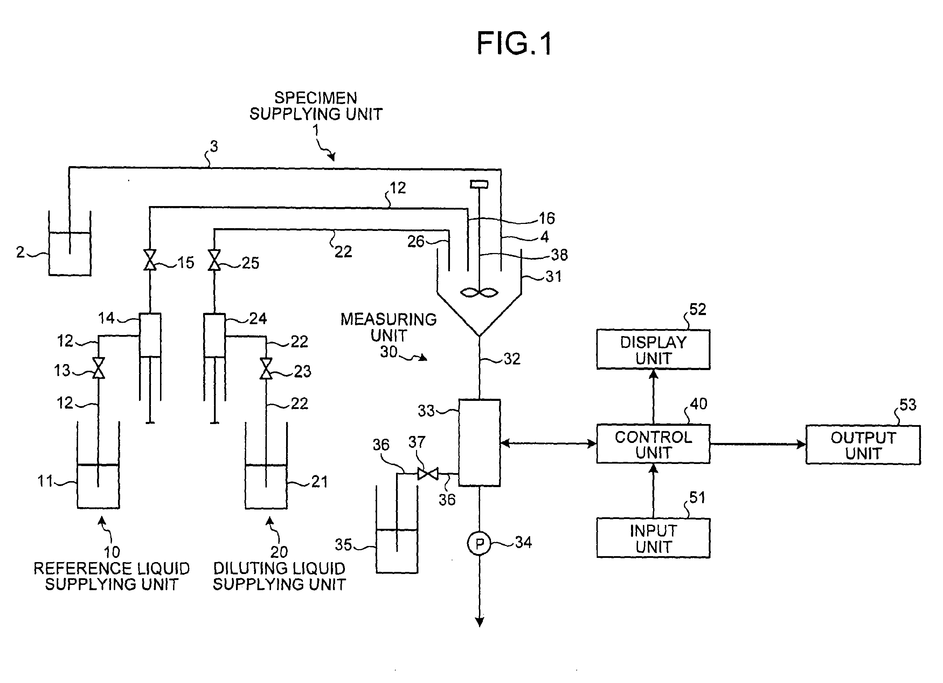

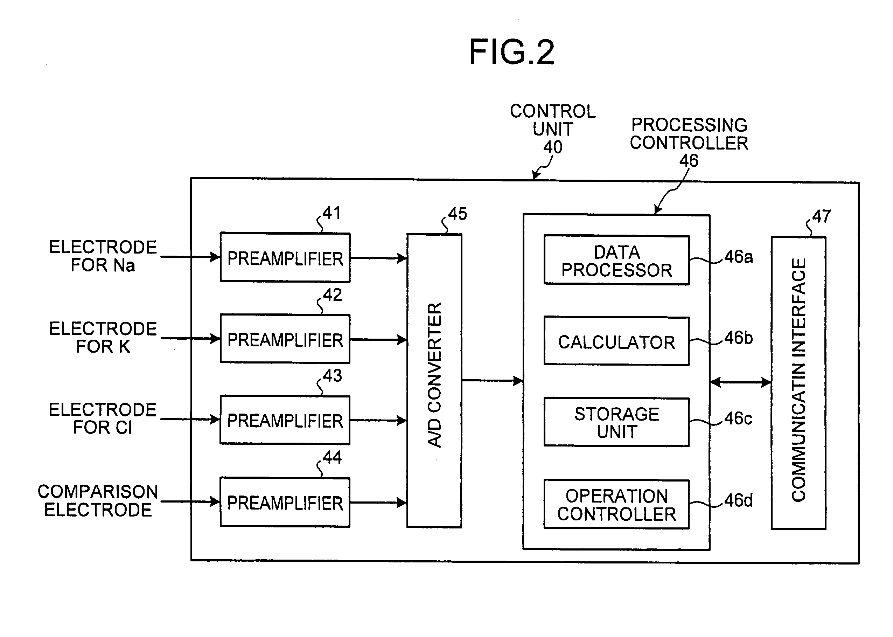

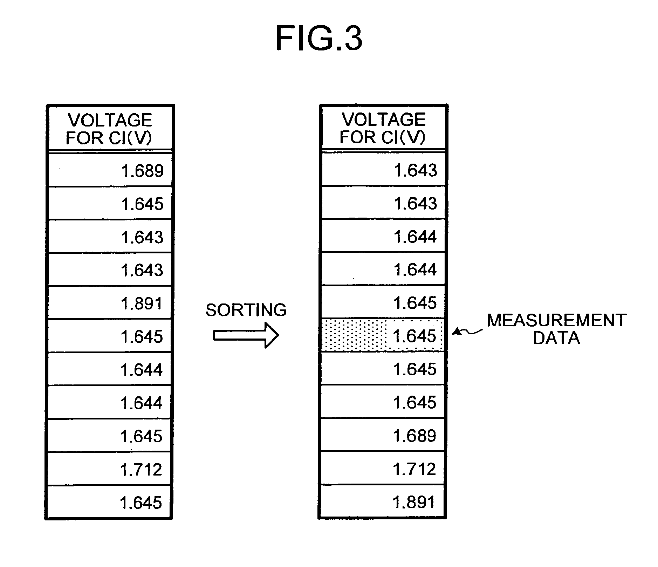

Exemplary embodiments of an electrolyte analyzing apparatus and a measurement data processing method of the same according to the present invention will be explained in detail below with reference to the accompanying drawings. FIG. 1 is a schematic diagram of a structure of an electrolyte analyzing apparatus according to an embodiment of the present invention. FIG. 2 is a detail view of a control unit of the electrolyte analyzing apparatus shown in FIG. 1. FIG. 3 shows a measurement data processing method in which multiple pieces of digital data is sorted depending on data values and a median of the digital data is treated as measurement data of an electrolyte by a data processor of the control unit shown in FIG. 2.

The electrolyte analyzing apparatus includes a specimen supplying unit 1, a reference liquid supplying unit 10, a diluting liquid supplying unit 20, a measuring unit 30, and a control unit 40 as shown in FIG. 1.

The specimen supplying unit 1 transfers a specimen kept in a specimen vessel 2 via a duct 3 and dispenses a predetermined amount of the specimen into a dilution tank 31 from a specimen dispensing nozzle 4.

The reference liquid supplying unit 10 dispenses a reference liquid in a reference liquid vessel 11 into the dilution tank 31 from a dispensing nozzle 16 via a duct 12. The reference liquid supplying unit 10 is provided with an electromagnetic valve 13, a syringe pump 14, and an electromagnetic valve 15 in the duct 12 between the reference liquid vessel 11 and the dispensing nozzle 16.

The diluting liquid supplying unit 20 dispenses the reference liquid in a diluting liquid vessel 21 into the dilution tank 31 from a dispensing nozzle 26 via a duct 22. The diluting liquid supplying unit 20 is provided with an electromagnetic valve 23, a syringe pump 24, and an electromagnetic valve 25 in the duct 22 between the diluting liquid vessel 21 and the dispensing nozzle 26.

The measuring unit 30 is provided with a measurement cell 33 and a pump 34 in a duct 32 connected to the dilution tank 31. A duct 36 which is provided with an electromagnetic valve 37 and guides a comparison electrode liquid from a comparison electrode liquid vessel 35 is connected to a bottom part of the measurement cell 33. Here, a plurality of ion selective electrodes are present in the measurement cell 33 for each electrolyte as a measurement subject. For example, three kinds of ion selective electrodes for respectively measuring ion concentrations of sodium (Na), potassium (K), and chlorine (Cl) and the comparison electrode are housed in the measurement cell 33. Here, the comparison electrode performs a measurement alternately whenever each ion selective electrode completes a measurement in the three kinds of ion selective electrodes and the comparison electrode. In addition, the specimen and a liquid such as the diluting liquid dispensed into the dilution tank 31 are stirred homogeneously by a stirring bar 38.

The control unit 40, as well as controlling an operation of each unit constituting the electrolyte analyzing apparatus, processes data concerning each ion concentration output from the measurement cell 33 and calculates a concentration of the electrolyte. The control unit 40 has preamplifiers 41 to 44, an A/D converter 45, a processing controller 46, and a communication interface 47 as shown in FIG. 2. The control unit 40 is connected to an input unit 51 such as a keyboard, a display unit 52 such as a display panel, and an output unit 53 such as a printer as shown in FIG. 1.

The preamplifiers 41 to 44 respectively amplify output signals (voltage values) output from the three kinds of ion selective electrodes and the comparison electrode to output to the A/D converter 45. The A/D converter 45, having a multiplexer, is a delta-sigma A/D converter which sequentially receives, by switching with the multiplexer, analogue data respectively output from the preamplifiers 41 to 44 in a time division manner and converts the analogue data into digital data.

The processing controller 46 has a data processor 46a, a calculator 46b, a storage unit 46c, and an operation controller 46d as shown in FIG. 2, and a calculation processor (CPU) is used. The data processor 46a sorts multiple pieces of digital data output from the A/D converter 45 for each ion concentration of Na, K, and Cl, and outputs a median of the sorted multiple pieces of digital data to the calculator 46b as measurement data of the electrolyte. The calculator 46b uses a calibration curve measured in advance for each ion concentration of Na, K, and Cl to calculate the concentration of the electrolyte contained in the specimen based on the received measurement data (the median of the digital data). The storage unit 46c stores data of the calibration curve, measured values related to the specimen, and the like. The operation controller 46d controls an operation of each unit described above constituting the electrolyte analyzing apparatus.

The communication interface 47 serves as an interface which transfers data and the like by connecting the electrolyte analyzing apparatus to another electrolyte analyzing apparatus, or by connecting the electrolyte analyzing apparatus to an implementer's host computer via an online network.

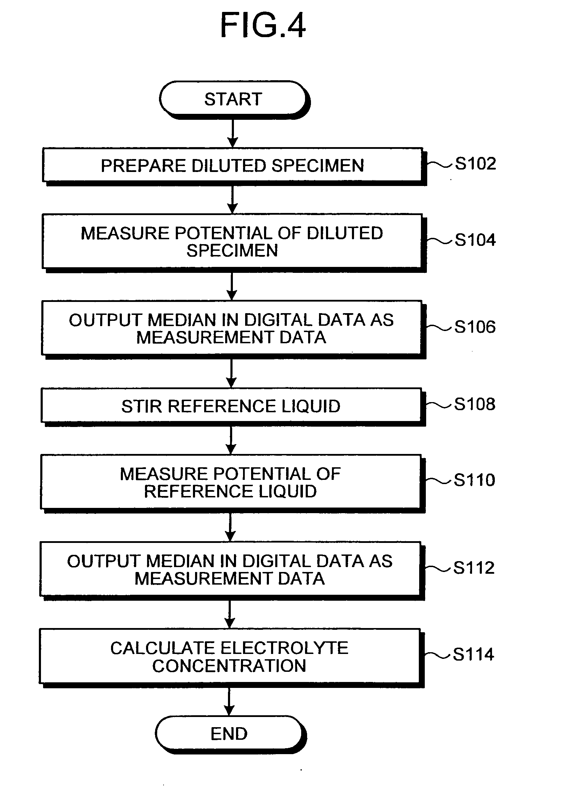

The electrolyte analyzing apparatus constituted in the manner described above measures each ion concentration of Na, K, and Cl in a way to be explained below and measures the concentration of the electrolyte contained in the specimen under the control by the control unit 40. To make the explanation short here, a measurement data processing method based on the Cl ion concentration, for example, will be explained below with reference to the flowchart shown in FIG. 4.

First, the control unit 40 dispenses the specimen from the specimen dispensing nozzle 4 and the diluting liquid from the dispensing nozzle 26 individually to the dilution tank 31, and prepares a diluted specimen of a predetermined concentration by stirring with the stirring bar 38 (step S102). Next, the control unit 40 drives the pump 34 to absorb the diluted specimen in the dilution tank 31 into the measurement cell 33 and measures the potential of the diluted specimen by each of the Cl electrode and the comparison electrode (step S104).

On this occasion, analogue voltage values for the Cl ion concentration output from the Cl electrode and the comparison electrode are amplified by the preamplifiers 43 and 44, then input to the A/D converter 45 via a time division switching by the multiplexer, and converted to digital voltage values, respectively.

The control unit 40 uses the data processor 46a to output a median of the multiple voltage values (digital data) output from the A/D converter 45 with respect to the Cl electrode and the comparison electrode to the calculator 46b as measurement data of the diluted specimen (step S106).

Besides, the control unit 40 drives the pump 34 to dispose of the diluted specimen in the dilution tank 31 and the measurement cell 33 after measuring the diluted specimen, and drives the syringe pump 24 to dispense the diluting liquid and clean the dilution tank 31 and the measurement cell 33.

Next, the control unit 40 drives the syringe pump 14 to dispense the reference liquid in the reference liquid vessel 11 into the dilution tank 31 from the dispensing nozzle 16, and stirs the reference liquid by the stirring bar 38 (step S108). Then, the control unit 40 drives the pump 34 to absorb the reference liquid in the dilution tank 31 into the measurement cell 33 and measures the potential of the reference liquid by each of the Cl electrode and the comparison electrode (step S110). On this occasion, analogue voltage values for the reference liquid output from the Cl electrode and the comparison electrode are amplified by the preamplifiers 43 and 44, then input to the A/D converter 45 via a time division switching by the multiplexer, and converted to digital voltage values, respectively, similarly to the case of the diluted specimen.

The control unit 40 uses the data processor 46a to output a median of the multiple voltage values (digital data) output from the A/D converter 45 with respect to the Cl electrode and the comparison electrode to the calculator 46b as measurement data of the reference liquid (step S112).

After that, the control unit 40 uses the calculator 46b to calculate the electrolyte concentration attributed to the Cl ion contained in the specimen based on the measurement data of the diluted specimen and the reference liquid measured by the Cl electrode, and the measurement data of the diluted specimen and the reference liquid measured by the comparison electrode, both measurement data being output by the data processor 46a (step S114).

On this occasion, as shown in FIG. 3 for example, the data processor 46a sorts multiple voltage values (digital data) for the Cl ion concentration output from the A/D converter 45, and outputs a median of the sorted multiple pieces of digital data to the calculator 46b as measurement data of the electrolyte. The same applies to multiple voltage values (digital data) for the reference liquid.

Here, though having a high degree of precision compared to a conventionally used flash A/D converter because of its delta-sigma system, the A/D converter 45 takes a sampling time longer by about three digits. Because of this, the A/D converter 45 has a higher possibility that a pulsing noise which arises unexpectedly gets mixed in the measurement data than the flash A/D converter. The A/D converter 45 receives signals respectively output from the plural electrodes via the switching by the multiplexer. For this reason, the A/D converter 45 results in having one fourth of the number of pieces of data generated by converting the signals input from the electrodes, compared to a case of arranging the A/D converter 45 for each electrode.

Therefore, it is possible to avoid an influence of an unexpected pulsing noise by using a median of the multiple pieces of digital data as measurement data of the electrolyte, compared to a case of using another value, for example, an average value or a mode value of the multiple pieces of digital data. In addition, since the A/D converter 45 outputs highly precise digital data compared to the conventionally-used flash A/D converter, it is possible to obtain measurement data which is close to a right value even with less number of pieces of digital data by using a median as measurement data. Therefore, the electrolyte analyzing apparatus according to the present invention is provided with the data processor 46a which outputs a median of multiple pieces of digital data to the calculator 46b as measurement data of the electrolyte.

The electrolyte analyzing apparatus according to the present invention may be equipped to an automatic analyzing apparatus which performs a biochemical analysis and an immunologic analysis, and such a usage enables the automatic analyzing apparatus to be used for various purposes.

Besides, as best modes for carrying out the invention, the electrolyte analyzing apparatus described above uses the A/D converter 45 of delta-sigma conversion system and the data processor 46a which treats a median of multiple pieces of digital data as measurement data of the electrolyte. However, needless to say, the electrolyte analyzing apparatus according to the present invention, as long as the data processor 46a treats the median of multiple pieces of digital data as measurement data of the electrolyte, may use the conventional flash A/D converter instead of the A/D converter 45 of delta-sigma conversion system.

In addition, though each of the sodium (Na) ion, the potassium (K) ion, and the chlorine (Cl) ion is mentioned as a subject to be measured in the measurement cell 33 in the electrolyte analyzing apparatus described above, ion selective electrodes which respectively measure ion concentrations of, for example, hydrogen (H), calcium (Ca), lithium (Li), and ammonium (NH4) other than the above-mentioned ions can be provided.

### INDUSTRIAL APPLICABILITY

As described above, the electrolyte analyzing apparatus and the measurement data processing method according to the present invention are useful for precisely measuring a specimen without a waste of time while avoiding an unexpected irregular noise contained in measurement data of the specimen.

## Claims

1. An electrolyte analyzing apparatus, comprising:
an A/D converter which converts analogue data output from an ion selective electrode into digital data;
a data processor which calculates a median of multiple pieces of the digital data converted from multiple pieces of the analogue data output from the ion selective electrode and outputs the median as measurement data of an electrolyte; and
a calculator which calculates a concentration of the electrolyte contained in a specimen based on the measurement data of the electrolyte output from the data processor.

2. The electrolyte analyzing apparatus according to claim 1, wherein the A/D converter employs a delta-sigma conversion system.

3. The electrolyte analyzing apparatus according to claim 1 or 2, wherein
the ion selective electrode is provided in plural number for each electrolyte as a measurement subject, and
the A/D converter receives the analogue data output from each ion selective electrode in a time division manner.

4. A measurement data processing method of an electrolyte analyzing apparatus which calculates a concentration of an electrolyte contained in a specimen based on measurement data converted from analogue data output from an ion selective electrode, wherein a median of multiple pieces of digital data converted from multiple pieces of the analogue data output from the ion selective electrode is treated as the measurement data of the electrolyte.

5. The measurement data processing method of the electrolyte analyzing apparatus according to claim 4, wherein the digital data is obtained by performing a delta-sigma A/D conversion on the analogue data.
